# EUROPEAN PATENT SPECIFICATION

(11) **EP 0 867 509 B1**
(45) Date of publication and mention of the grant of the patent: **14.08.2002**
(21) Application number: 98300827.7
(22) Date of filing: 04.02.1998
(51) Int. Cl.: C12N 15/12, C07K 14/715, C07K 16/28, C12N 5/10, C12Q 1/68, G01N 33/68, A61K 38/17

(54) **Method to identify substances which stimulate or inhibit the binding of TL2 (TRAIL) to TR5.**
Verfahren zur Identifizierung von Substanzen, die die Bindung von TL2 (TRAIL) an TR5 hemmen oder stimulieren.
Méthode pour l'identification de substances qui stimulent ou inhibent la liaison de TL2 (TRAIL) à TR5.

(30) Priority: 05.02.1997 US 795910; 28.07.1997 US 901469
(43) Date of publication of application: 30.09.1998
(73) Proprietor: SMITHKLINE BEECHAM CORPORATION, Philadelphia Pennsylvania 19103 (US)
(72) Inventor: Young, Peter, R., SmithKline Beecham Pharm., King of Prussia, Pennsylvania 19406 (US); Tan, Kong B., SmithKline Beecham Pharm., King of Prussia, Pennsylvania 19406 (US); Truneh, Alemseged, SmithKline Beecham Pharm., King of Prussia, Pennsylvania 19406 (US); Lyn, Sally Doreen P., SmithKline Beecham Pharm., King of Prussia, Pennsylvania 19406 (US)
(74) Representative: Connell, Anthony Christopher

(56) References cited:
- WO-A-98/30693
- HILLIER L ET AL: "Homo sapiens cDNA clone 504745 5' (accession number AA150849)" EMBL SEQUENCE DATABASE, 15 December 1996, XP002076202 Heidelberg, Germany
- HILLIER L ET AL: "Homo sapiens cDNA clone 504745 3' (accession number AA150541)" EMBL SEQUENCE DATABASE, 15 December 1996, XP002076203 Heidelberg, Germany
- PAN G ET AL: "AN ANTAGONIST DECOY RECEPTOR AND A DEATH DOMAIN-CONTAINING RECEPTOR FOR TRAIL" SCIENCE, vol. 277, 8 August 1997, pages 815-818, XP002065147
- SHERIDAN J P ET AL: "CONTROL OF TRAIL-INDUCED APOPTOSIS BY A FAMILY OF SIGNALING AND DECOY RECEPTORS" SCIENCE, vol. 277, 8 August 1997, pages 818-821, XP002065023
- PITTI R M ET AL: "INDUCTION OF APOPTOSIS BY APO-2 LIGAND, A NEW MEMBER OF THE TUMOR NECROSIS FACTOR CYTOKINE FAMILY" JOURNAL OF BIOLOGICAL CHEMISTRY, vol. 271, no. 22, 31 May 1996, pages 12687-12690, XP002065021
- Chinnaiyan A. M. et. al.: "Signal transduction by DR3, a Death Domain-containing receptor related to TNFR-1 and CD95", Science, vol. 274, no. 5289, 8 November 1996, pages 990-992
- Bucher P. et. al. : "A flexible motif search technique based on generalized profiles", Computers Chemistry, vol.20, no 1, 1996, pages 3-23.

## Description

### FIELD OF INVENTION

The polynucleotides and polypeptides of the present invention relate to Tumor necrosis factor receptor (TNF-R) related protein, hereinafter referred to as TR5. The invention is directed towards a method for identifying substances which stimulate or inhibit the binding of the TL2 ligand of SEQ ID NO: 3 to a TR5 polypeptide.

### BACKGROUND OF THE INVENTION

Many biological actions, for instance, response to certain stimuli and natural biological processes, are controlled by factors, such as cytokines. Many cytokines act through receptors by engaging the receptor and producing an intracellular response.

For example, tumor necrosis factors (TNF) alpha and beta are cytokines which act through TNF receptors to regulate numerous biological processes, including protection against infection and induction of shock and inflammatory disease. The TNF molecules belong to the "TNF-ligand" superfamily, and act together with their receptors or counter-ligands, the "TNF-receptor" superfamily. So far, nine members of the TNF ligand superfamily have been identified and ten members of the TNF-receptor superfamily have been characterized.

Among the ligands there are included TNF-a, lymphotoxin-a (LT-a, also known as TNF-b), LT-b (found in complex heterotrimer LT-a2-b), FasL, CD40L, CD27L, CD30L, 4-1BBL, OX40L and nerve growth factor (NGF)). The superfamily of TNF receptors includes the p55TNF receptor, p75TNF receptor, TNF receptor-related protein, FAS antigen or APO-1, CD40, CD27, CD30, 4-1BB, OX40, low affinity p75 and NGF-receptor (Meager, A., Biologicals, 22:291-295 (1994)).

Many members of the TNF-ligand superfamily are expressed by activated T-cells, implying that they are necessary for T-cell interactions with other cell types which underlie cell ontogeny and functions. (Meager, A., supra).

Considerable insight into the essential functions of several members of the TNF receptor family has been gained from the identification and creation of mutants that abolish the expression of these proteins. For example, naturally occurring mutations in the FAS antigen and its ligand cause lymphoproliferative disease (Watanabe-Fukunaga, R., et al., Nature 356:314 (1992)), perhaps reflecting a failure of programmed cell death. Mutations of the CD40 ligand cause an X-linked immunodeficiency state characterized by high levels of immunoglubulin M and low levels of immunoglobulin G in plasma, indicating faulty T-cell-dependent B-cell activation (Allen, R.C. et al., Science 259:990 (1993)). Targeted mutations of the low affinity nerve growth factor receptor cause a disorder characterized by faulty sensory innovation of peripheral structures (Lee, K.F. et al, Cell 69:737 (1992)).

TNF and LT-a are capable of binding to two TNF receptors (the 55-and 75-kd TNF receptors). A large number of biological effects elicited by TNF and LT-a, acting through their receptors, include hemorrhagic necrosis of transplanted tumors, cytotoxicity, a role in endotoxic shock, inflammation, immunoregulation, proliferation and anti-viral responses, as well as protection against the deleterious effects of ionizing radiation. TNF and LT-a are involved in the pathogenesis of a wide range of diseases, including endotoxic shock, cerebral malaria, tumors, autoimmuine disease, AIDS and graft-host rejection (Beutler, B. and Von Huffel, C., Science 264:667-668 (1994)). Mutations in the p55 Receptor cause increased susceptibility to microbial infection.

Moreover, an about 80 amino acid domain near the C-terminus of TNFR1 (P55) and Fas was reported as the "death domain," which is responsible for transducing signals for programmed cell death (Tartaglia et al., Cell 74:845 (1993)).

The effects of TNF family ligands and TNF family receptors are varied and influence numerous functions, both normal and abnormal, in the biological processes of the mammalian system. There is a clear need, therefore, for identification and characterization of such receptors and ligands that influence biological activity, both normally and in disease states.

### SUMMARY OF THE INVENTION

In one aspect, the invention relates to a method for identifying compounds which stimulate or inhibit the binding of the TL2 ligand to TR5 polypeptides.

### BRIEF DESCRIPTION OF THE DRAWINGS

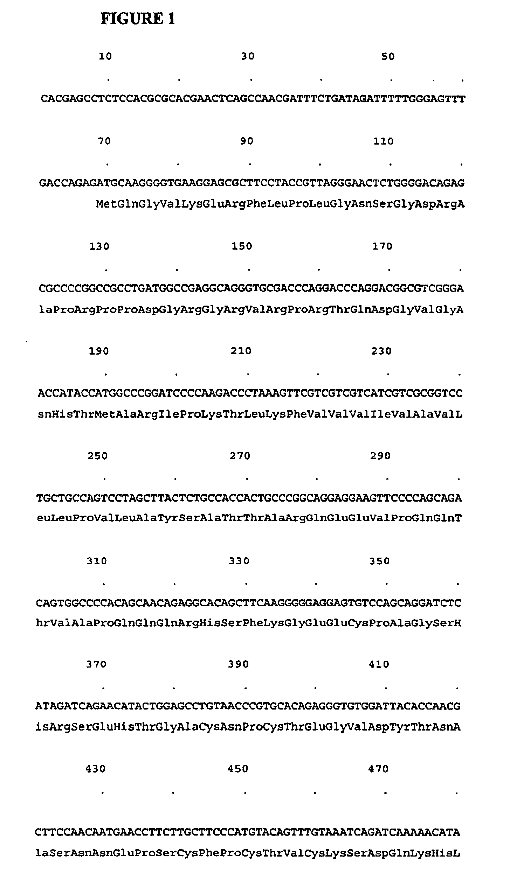

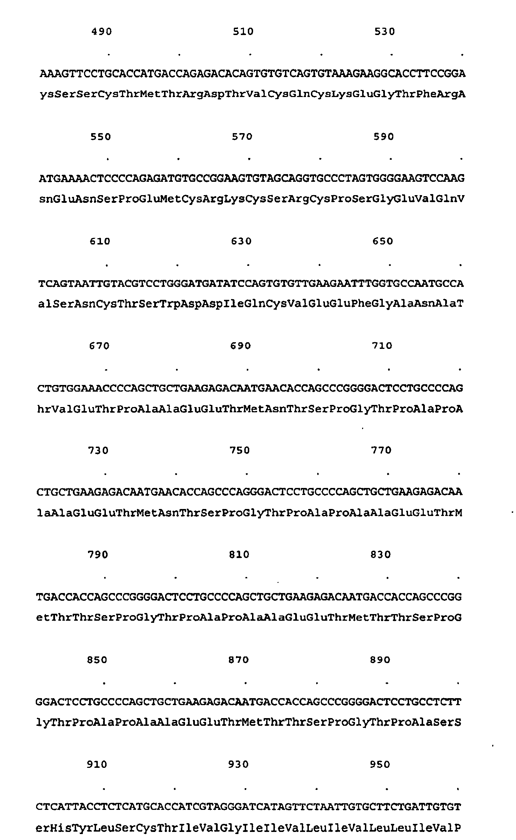

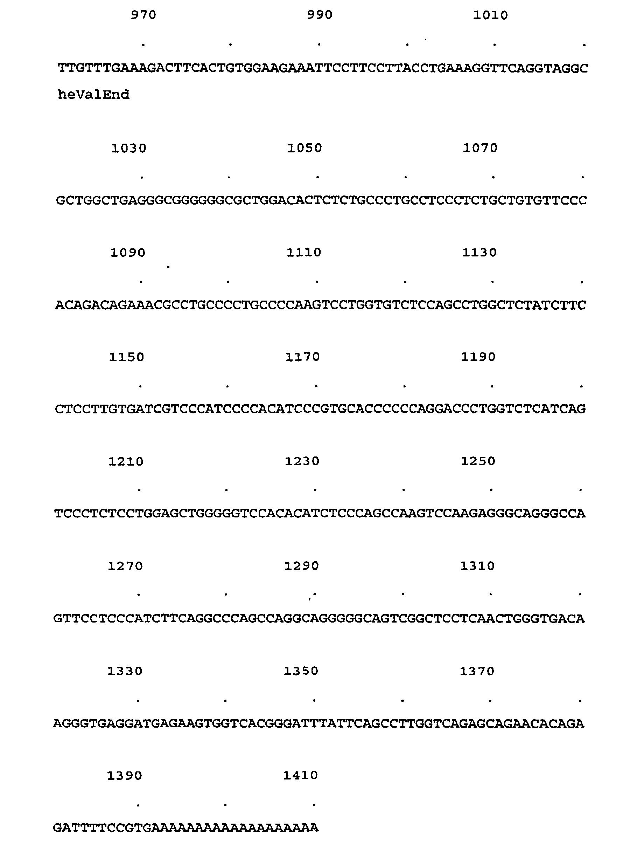

Figure 1 shows the nucleotide and deduced amino acid sequence of human TR5.

### DESCRIPTION OF THE INVENTION

### Definitions

The following definitions are provided to facilitate understanding of certain terms used frequently herein.

"TR5" refers generally to a polypeptide having the amino acid sequence set forth in SEQ ID NO:2 or an allelic variant thereof.

"TR5 activity or TR5 polypeptide activity" or "biological activity of the TR5 or TR5 polypeptide" refers to the metabolic or physiologic function of said TR5 including similar activities or improved activities or these activities with decreased undesirable side-effects. Also included are antigenic and immunogenic activities of said TR5.

"TR5 polypeptides" refers to polypeptides with amino acid sequences sufficiently similar to TR5 sequences, preferably exhibiting at least one biological activity of the TR5.

"TR5 gene" refers to a polynucleotide having the nucleotide sequence set forth in SEQ ID NO:1 or allelic variants thereof and/or their complements.

"TR5 polynucleotides" refers to polynucleotides containing a nucleotide sequence which encodes a TR5 polypeptide or fragment thereof, or a nucleotide sequence which has at least 80% identity to a nucleotide sequence encoding the polypeptide of SEQ ID NO:2 or the corresponding fragment thereof, or a nucleotide sequence which has sufficient identity to a nucleotide sequence contained in SEQ ID NO:1 to hybridize under conditions useable for amplification or for use as a probe or marker.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, crosslinking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2^{nd} Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter et al., "Analysis for protein modifications and nonprotein cofactors", Meth Enzymol (1990) 182:626-646 and Rattan et al., "Protein Synthesis: Posttranslational Modifications and Aging", Ann NY Acad Sci (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" per se has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., SIAM J Applied Math (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., SIAM J Applied Math (1988) 48:1073. Methods to determine identity and similarity are codified in computer programs. Preferred computer program methods to determine identity and similarity between two sequences include, but are not limited to, GCS program package (Devereux, J., et al., Nucleic Acids Research (1984) 12(1):387), BLASTP, BLASTN, FASTA (Atschul, S.F. et al., J Molec Biol (1990) 215:403).

### TR5 Polypeptides

The TR5 polypeptides utilised in the present invention include the polypeptide of SEQ ID NO:2 (in particular the mature polypeptide) as well as TR5 polypeptides and which have at least 80% identity to the polypeptide of SEQ ID NO:2 or the relevant portion and more preferably at least 85% identity, and still more preferably at least 90% identity, and even still more preferably at least 95% identity to SEQ ID NO: 2.

The TR5 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Thus, the polypeptides utilised in the invention include polypeptides having an amino acid sequence at least 80% identical to that of SEQ ID NO:2 or fragments thereof with at least 80% identity to the corresponding fragment of SEQ ID NO:2. Preferably, all of these polypeptides retain the biological activity of the TR5, including antigenic activity. Included in this group are variants of the defined sequence and fragments. Preferred variants are those that vary from the referents by conservative amino acid substitutions i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The TR5 polypeptides utilised in the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

TR5 utilised in the invention is structurally related to other proteins of the Tumor necrosis factor receptor (TNF-R), as shown by the results of sequencing the cDNA encoding human TR5. The cDNA sequence contains an open reading frame encoding a protein of 299 amino acids with a deduced molecular weight of 31.8 kDa. TR5 of Figure 1 (SEQ ID NO:2) has about 25.6% identity (using BESTFIT (from GCG suite of Programs)) in 274 amino acid residues with Human low affinity Nerve Growth Factor receptor (LNGF-R) (SWISSPROT Accession: NGFR_HUMAN; D. Johnson et al., Cell 47:545-554 (1986)).. Furthermore, TR5 (SEQ ID NO:2) is 22.4% identical to low affinity rat Nerve Growth Factor Receptor over 281 amino acid residues (SWISSPROT Accession: NGFR_RAT; M.J. Radeke et al., Nature 325:593-597 (1987)). TR5 gene of Figure 1 (SEQ ID NO:1) has about 89% identity (using BLAST) in 37 nucleotide residues with H. Sapiens CpG island DNA genomic MseI fragment (Genbank Accession Z54987; S.H Cross et al., Nature Genet. 6:236-244 (1994)). Furthermore, TR5 is 52% identical to O. cuniculus KAP4C mRNA over 195 nucleotide base residues (Genbank Accession X80035; B.C. Powell et al., Differentiation 58:227-232 (1995))

One polynucleotide encoding TR5 may be obtained using standard cloning and screening, from a cDNA library derived from mRNA in cells of prostate, endothelial cells, IL-1beta treated smooth muscle cells, fetal liver spleen cells, and pregnant uterususing the expressed sequence tag (EST) analysis (Adams, M.D., et al. Science (1991) 252:1651-1656; Adams, M.D. et al., Nature, (1992) 355:632-634; Adams, M.D., et al., Nature (1995) 377 Supp:3-174). Polynucleotides of the invention can also be obtained from natural sources such as genomic DNA libraries or can be synthesized using well known and commercially available techniques.

Thus, the nucleotide sequence encoding TR5 polypeptides may be identical over its entire length to the coding sequence in Figure 1 (SEQ ID NO:1), or may be a degenerate form of this nucleotide sequence encoding the polypeptide of SEQ ID NO:2, or may be highly identical to a nucleotide sequence that encodes the polypeptide of SEQ ID NO:2. Preferably, the polynucleotides of the invention contain a nucleotide sequence that is highly identical, at least 80% identical, with a nucleotide sequence encoding a TR5 polypeptide, or at least 80% identical with the encoding nucleotide sequence set forth in Figure 1 (SEQ ID NO:1), or at least 80% identical to a nucleotide sequence encoding the polypeptide of SEQ ID NO:2.

When TR5 polynucleotides are used for the recombinant production of TR5 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz et al., Proc Natl Acad Sci USA (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Among particularly preferred TR5 polynucleotides are polynucleotides encoding TR5 polypeptides having the amino acid sequence of set out in Figure 1 (SEQ ID NO:2) and variants thereof.

Further preferred embodiments of TR5 polynucleotides are polynucleotides encoding TR5 variants that have the amino acid sequence of the TR5 polypeptide of Figure 1 (SEQ ID NO:2) in which several, 5-10, 1-5, 1-3, 1-2 or 1 amino acid residues are substituted, deleted or added, in any combination.

Further preferred embodiments of TR5 polynucleotides are polynucleotides that are at least 80% identical over their entire length to a polynucleotide encoding the TR5 polypeptide having the amino acid sequence set out in Figure 1 (SEQ ID NO:2), and polynucleotides which are complementary to such polynucleotides. In this regard, polynucleotides at least 80% identical over their entire length to the same are particularly preferred, and those with at least 90% are especially preferred. Furthermore, those with at least 97% are highly preferred and those with at least 98-99% are most highly preferred, with at least 99% being the most preferred.

TR5 polynucleotides which are sufficiently identical to a nucleotide sequence contained in SEQ ID NO:1, may be used as hybridization probes for cDNA and genomic DNA, to isolate full-length cDNAs and genomic clones encoding TR5 polypeptide and to isolate cDNA and genomic clones of other genes that have a high sequence similarity to the TR5 gene. Such hybridization techniques are known to those of skill in the art. Typically these nucleotide sequences are 70% identical, preferably 80% identical, more preferably 90% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

### Screening Assays

We have now discovered that TL2 of SEQ ID NO: 3 (otherwise known as TRAIL, TWiley SR, et al., Immunity (6):673-682 (1995)) is a ligand of TR5. Thus, TR5 polypeptide, and one of its ligands, TL2 may be employed in a screening process for compounds which bind the receptor, or its ligand, and which activate (agonists) or inhibit activation of (antagonists) the receptor polypeptide of the present invention, or its ligand TL2. Thus, polypeptides of the invention may be used to assess the binding of small molecule substrates and ligands in, for example, cells, cell-free preparations, chemical libraries, and natural product mixtures. These substrates and ligands may be natural substrates and ligands or may be structural or functional mimetics. See Coligan et al., Current Protocols in Immunology 1(2):Chapter 5 (1991).

TR5 polypeptides are responsible for many biological functions, including many pathologies. Accordingly, it is desirable to find compounds and drugs which stimulate TR5 on the one hand and which can inhibit the function of TR5 or remove TR5 expressing cells on the other hand. Antagonists, or agents which remove TR5 expressing cells, may be employed for a variety of therapeutic and prophylactic purposes for such conditions as chronic and acute inflammation, arthritis, septicemia, autoimmune diseases (e.g. inflammatory bowel disease, psoriasis), transplant rejection, graft vs. host disease, infection, stroke, ischemia, acute respiratory disease syndrome, restenosis, brain injury, AIDS, Bone diseases, cancer (e.g. lymphoproliferative disorders), atheroschlerosis, and Alzheimers disease. Agonists can be employed for therapeutic and prophylactic purposes for such conditions responsive to activation of T cells and other components of the immune system, such as for treatment of cancer and AIDS. However, agonists can also be employed for inappropriate stimulation of T cells and other components of the immune system which leads to down modulation of immune activity with therapeutic or prophylactic application for conditions such , as chronic and acute inflammation, arthritis, septicemia, autoimmune diseases (e.g. inflammatory bowel disease, psoriasis), transplant rejection, graft vs. host disease, infection, stroke, ischemia, acute respiratory disease syndrome, restenosis, brain injury, , Bone diseases,, atheroschlerosis, and Alzheimers disease.

Candidate compounds may be identified using assays to detect compounds which inhibit binding of TL2 to TR5 in either cell-free or cell based assays. Suitable cell-free assays may be readily determined by one of skill in the art. For example, an ELISA format may be used in which purified TR5, or a purified derivative of TR5, containing the extracellular domain of TR5, is immobilized on a suitable surface, either directly or indirectly (e.g., via an antibody to TR5) and candidate compounds are identified by their ability to block binding of purified TL2 to TR5. The binding of TL2 to TR5 could be detected by using a label directly or indirectly associated with TL2. Suitable detection systems include the streptavidin horseradish peroxidase conjugate, or direct conjugation by a tag, e.g., fluorescein. Conversely, purified TL2 may be immobilized on a suitable surface, and candidate compounds identified by their ability to block binding of purified TR5 to TL2. The binding of TR5 to TL2 could be detected by using a label directly or indirectly associated with TR5. Many other assay formats are possible that use the TR5 protein and its ligands. Suitable cell based assays may be readily determined by one of skill in the art. In general, such screening procedures involve producing appropriate cells which express the receptor polypeptide of the present invention on the surface thereof. Such cells include cells from mammals, yeast, Drosophila or E. coli. Cells expressing the receptor (or cell membrane containing the expressed receptor) are then contacted with a known ligand, such as TL2, or test compound to observe binding, or stimulation or inhibition of a functional response. The assays may simply test binding of a candidate compound wherein adherence to the cells bearing the receptor is detected by means of a label directly or indirectly associated with the candidate compound or in an assay involving competition with a labeled competitor, such as the ligand TL2. Further, these assays may test whether the candidate compound results in a signal generated by activation of the receptor or its ligand (e.g. TL2) using detection systems appropriate to the cells bearing the receptor or its ligand and fusion proteins thereof at their surfaces. Typical fusion partners include fusing the extracellular domain of the receptor or ligand with the intracellular tyrosine kinase domain of a second receptor. Inhibitors of activation are generally assayed in the presence of a known agonist, such as the ligand TL2, and the effect on activation by the agonist by the presence of the candidate compound is observed. Standard methods for conducting such screening assays are well understood in the art.

Examples of potential TR5 antagonists include antibodies or, in some cases, oligonucleotides or proteins which are closely related to the ligand of the TR5, e.g., a fragment of the ligand TL2, or small molecules which bind to the receptor, or its ligand, but do not elicit a response, so that the activity of the receptor is prevented. Examples of potential TR5 agonists include antibodies that bind to TR5, its ligand, such as TL2, or derivatives thereof, and small molecules that bind to TR5. These agonists will elicit a response mimicking all or part of the response induced by contacting the native ligand.

Alternatively, TR5 may be expressed as a soluble protein, including versions which fuse all or part of TR5 with a convenient partner peptide for which detection reagents are available, eg TR5-IgG fusions, and used in a solid state or solution phase binding assay. For example, the soluble TR5 can be used to detect agonist or antagonist binding directly through changes that can be detected experimentally, eg surface plasmon resonance, nuclear magnetic resonance spectrometry, sedimentation, calorimetry. The soluble TR5 can be used to detect agonist or antagonist binding indirectly by looking for competition of the candidate agonist or antagonist with a ligand whose binding can be detected. Ligand detection methods include antibody recognition, modification of the ligand via radioactive labeling, chemical modification (eg biotinylation), fusion to an epitope tag. Methods include ELISA based assays, immunoprecipitation and scintillation proximity.

Assays similar to those described above using soluble or membrane bound TR5 may also be used to identify and purify the natural ligand(s) of TR5. These ligands may be agonists or antagonists of the receptor.

Examples of potential TR5 polypeptide antagonists include antibodies or, in some cases, oligonucleotides or proteins which are closely related to the ligands, substrates, receptors, etc., as the case may be, of the TR5 polypeptide, e.g., a fragment of the ligands, substrates, receptors, or small molecules which bind to the polypeptide of the present invention but do not elicit a response, so that the activity of the polypeptide is prevented.

The TR5 cDNA, protein and antibodies to the protein may also be used to configure assays for detetcting the effect of added compounds on the production of TR5 mRNA and protein in cells. For example, an ELISA may be constructed for measuring secreted or cell associated levels of TR5 protein using monoclonal and polyclonal antibodies by standard methods known in the art, and this can be used to discover agents (i.e. antagonists or agonists) which may inhibit or enhance the production of TR5 from suitably manipulated cells or tissues.

### Examples and Descriptions

The examples below are carried out using standard techniques, which are well known and routine to those of skill in the art, except where otherwise described in detail. The examples illustrate, but do not limit the invention.

### Description 1

An EST (EST# 213397; Project ID HPRCB54) derived from a cDNA library made from human prostate and deposited in a commercial EST database was found to have sequence similarity to the human TNF receptor. A search through several overlapping ESTs indicated that this represented the 5' most EST of the assembly and so it was completely sequenced. Analysis of the 1410 nucleotide cDNA sequence indicated that it encoded a complete open reading frame for a novel member of the TNF receptor superfamily and was named TR5. The predicted protein is 300 amino acids long, and contains an amino-terminal hydrophobic signal sequence required for secretion. Cleavage is predicted to occur at the amino acid #65/66 junction. This means that the signal sequence is amino acids 1-65 (or 41-65 if translation starts at the second Methionine at position 41), and the mature protein is amino acids 66-299 (ie 234 amino acids). Which of the initiation sites is used is not known. Furthermore, the protein contains a carboxy terminal hydrophobic region and lacks a cytoplasmic tail, which is characteristic of proteins which are cleaved and attached to the extracellular membrane surface through a glycosylphosphatylinositol (GPI) linkage. In addition to being found on the surface of cells, GPI-linked proteins can also be enzymatically released from cells as secreted proteins through cleavage of the GPI linkage. Comparison of TR5 with other TNF receptor family proteins indicates that it has at least two complete and one partial copy of the cysteine-rich repeats characteristic of the extracellular domains of this family. Between these repeat regions characteristic of the ligand binding domain of the TNF receptor family and the carboxy-terminus is a series of 5 repeats of an approximately 15 amino acid sequence rich in Glutamate (E), Threonine (T), Serine (S), Proline (P), Alanine (A) and Glycine (G). Such sequences are commonly associated with regions rich in O-glycosylation on Serine and Threonine. TR5 is most similar to the extracellular domain of the low affinity NGF receptor, which also has a membrane proximal O-glycosylation region.

### Chromosomal location.

The complete TR5 cDNA obtained above was searched against Genbank and identified an exact match between the 3' untranslated region (3' UTR) and a Sequence Tagged Site (STS) (Genbank Accession #G23178; human STS WI-11701). STS sequences are derived from PCR primer pairs often selected from the 3' UTRs of randomly sequenced cDNAs and subsequently used to map their chromosomal location via radiation hybrid mapping and other physical mapping methods. This STS had previously been chromosomally localized to a specific region of chromosome 8 between 8p12 and 8p22 (42-60cM from top of chromosome 8 between markers D8S298 and D8S505). No known TNF receptor family members are known to map to this chromosomal region.

### Northern blot of TR5.

Various tissues and cell lines were screened for mRNA expression by Northern blot. RNA was prepared from cells and cell lines using Tri-Reagent (Molecular Research Center Inc., Cincinnati, OH), run in denaturing agarose gels (Sambrook et al., Molecular Cloning: a laboratory manual, 2^{nd} Ed. Cold Spring Harbor Lab Press, NY (1989)) and transfered to Zeta-probe nylon membrane (Biorad, Hercules, CA.) via vacuum blotting in 25mM NaOh for 90 min. After neutralization for 5-10 minutes with 1M tris-HCl, pH 7.5 containing 3M NaCI, the blots were prehybridized with 50% formamide, 8% dextran sulfate, 6XSSPE, 0.1%SDS and 100mg/ml of sheared and dentured salmon sperm DNA for at least 30 min. At 42°C. cDNA probes were labeled with 32P-CTP by random priming (Statagene, La Jolla, CA), briefly denatured with 0.25M NaOH and added to the prehybridization solution. After a further incubation for at least 24h at 42°C, the blots were washed in high stringency conditions and exposed to X-ray film.

TR5 was expressed in resting CD19+ B lymphocytes and CD8+ and CD4+ T lymphocytes. However, RNA was not detectable 48 hours following activation by PHA+PMA of CD8+ and CD4+ T lymphocytes. Highest expression of TR5 message was seen in bone marrow. High expression was also detected in monocytes and aortic endothelial cells.

RNA was in KG1a hematopoietic cell line, in HL60 following DMSO treatment (which leads to granulocytic differentiation), and in U937 and THP1 cells following PMA treatment treatment (which leads to monocytic differentiation). RNA was also detectable in HeLa cells.

Multiple forms of RNA (4) were detected ranging in sizes from 1.6 to 5 kb. The major forms expressed in lymphocytes are 1.6 and 2.5 kb in sizes. The major forms expressed in endothelial cells are the 1.6, 3.5 and 5 kb RNAs.

## Claims

1. A method for identifying substances which stimulate or inhibit the binding of the TL2 ligand of SEQ ID NO:3 to a TR5 polypeptide which comprises an amino acid sequence that is at least 80% identical to the amino acid sequence contained in SEQ ID NO:2; said method comprising the steps of:
(a) contacting a candidate substance with said TR5 polypeptide in the presence of said TL2 ligand; and
(b) assessing the ability of said candidate substance to compete with the binding of said TL2 ligand to said TR5 polypeptide.

2. A method as claimed in claim 1 wherein said TL2 ligand is directly or indirectly associated with a label, such as streptavidin horseradish peroxidase conjugate, or fluorescein.

3. A method as claimed in claim 1 or claim 2 wherein said TR5 polypeptide is expressed on the surface of a host cell, or on a cell membrane, or is immobilised on a suitable surface.

4. A method as claimed in claim 1 or claim 2, wherein said TL2 ligand is immobilised on a suitable surface.

## Patentansprüche

1. Verfahren zur Identifizierung von Substanzen, die die Bindung des TL2-Liganden mit SEQ ID NO:3 an ein TR5-Polypeptid, das eine Aminosäuresequenz umfaßt, die zu mindestens 80% mit der in SEQ ID NO:2 enthaltenen Aminosäuresequenz identisch ist, stimulieren oder hemmen, wobei das Verfahren die folgenden Schritte umfaßt:
(a) Kontaktieren einer Kandidatensubstanz mit dem TR5-Polypeptid in Gegenwart des TL2-Liganden; und
(b) Beurteilen der Fähigkeit der Kandidatensubstanz, mit der Bindung des TL2-Liganden an das TR5-Polypeptid zu konkurrieren.

2. Verfahren nach Anspruch 1, wobei der TL2-Ligand direkt oder indirekt mit einer Markierung, wie z.B. Streptavidin-Meerretichperoxidase-Konjugat oder Fluorescein, verbunden ist.

3. Verfahren nach Anspruch 1 oder Anspruch 2, wobei das TR5-Polypeptid auf der Oberfläche einer Gastzelle oder auf einer Zellmembran exprimiert oder auf einer geeigneten Oberfläche immobilisiert ist.

4. Verfahren nach Anspruch 1 oder Anspruch 2, wobei der TL2-Ligand auf einer geeigneten Oberfläche immobilisiert ist.

## Revendications

1. Méthode pour identifier des substances qui stimulent ou inhibent la liaison du ligand TL2 de la SEQ ID N° 3 à un polypeptide TR5 qui comprend une séquence d'amino-acides qui est identique à au moins 80 % à la séquence d'amine-acides présente dans la SEQ ID N° 2, méthode comprenant les étapes consistant :
(a) à mettre en contact une substance candidate avec ledit polypeptide TR5 en présence dudit ligand TL2 ; et
(b) à déterminer l'aptitude de ladite substance candidate à entrer en compétition avec la liaison dudit ligand TL2 audit polypeptide TR5.

2. Méthode suivant la revendication 1, dans laquelle le ligand TL2 est associé directement ou indirectement à un marqueur, tel qu'un conjugué streptavidine-peroxydase de raifort ou la fluorescéine.

3. Méthode suivant la revendication 1 ou la revendication 2, dans laquelle le polypeptide TR5 est exprimé sur la surface d'une cellule hôte, ou sur une membrane cellulaire, ou bien est immobilisé sur une surface convenable.

4. Méthode suivant la revendication 1 ou la revendication 2, dans laquelle le ligand TL2 est immobilisé sur une surface convenable.
